Europäisches Patentamt

European Patent Office

Office européen des brevets

(11) Publication number: **0 449 427 A2**

# (12) EUROPEAN PATENT APPLICATION

(21) Application number: **91301585.5**

(22) Date of filing: **27.02.91**

(51) Int. Cl.⁵: **A61K 31/00, A61K 31/135, A61K 31/44, A61K 31/495, A61K 31/54**

(30) Priority: **28.02.90 ZA 901537**

(43) Date of publication of application:
**02.10.91 Bulletin 91/40**

(84) Designated Contracting States:
**AT BE CH DE DK ES FR GB GR IT LI LU NL SE**

(71) Applicant: **CLIDET NO 12 (PROPRIETARY) LIMITED**
**78 Fox Street**
**Johannesburg, Transvaal (ZA)**

(72) Inventor: **Pretorius, George Archibald Clark**
**114 Albert Drive, Northcliff**
**Johannesburg, Transvaal (ZA)**

(74) Representative: **Spencer, Graham Easdale et al**
**A.A. Thornton & CO Northumberland House**
**303-306, High Holborn**
**London WC1V 7LE (GB)**

(54) Use of an antihistamine in the treatment of alcohol intoxication.

㊵ An antihistamine, particularly diphenhydramine hydrochloride, for use in reducing the after-effects of over indulgence of alcohol. The antihistamine is administered to a person after the person has consumed alcohol.

EP 0 449 427 A2

# PHARMACEUTICAL COMPOSITION

## BACKGROUND OF THE INVENTION

This invention relates to a pharmaceutical composition.

Over indulgence at parties or dinners puts a strain on various systems of the body giving rise to a range of discomforts. For example, over indulgence of alcohol affects the liver which is then called on to work harder by having to break down the alcohol. There are compositions on the market which are designed to assist the liver in its metabolic function under these circumstances. One such preparation contains glucuronolactone, glucuronamide, ascorbic acid and caffeine.

Another preparation useful for the treatment of the effects of hangover or over indulgence is the product Essentiale which contains a number of vitamins.

## SUMMARY OF THE INVENTION

It has been found, and this forms the basis of the present invention, that antihistamines are useful in reducing the after-effects of over indulgence of alcohol. Thus, the invention provides, according to one aspect, the use of an antihistamine in reducing the after-effects of over indulgence of alcohol.

Further according to the invention, there is provided a composition, preferably for oral administration, containing an effective amount of an antihistamine to reduce the after effects of over indulgence of alcohol.

Still further according to the invention, there is provided an antihistamine for use in a method of manufacturing a pharmaceutical composition of the type described above.

Examples of suitable antihistamines are ethanolamines such as diphenhydramine and doxylamine, ethylenediamines such as pyrilamine, alkylamines such as chlorpheniramine, piperazines such as chlorcyclizine and phenothiazines such as promethazine.

The antihistamine may be provided in the form of the base or in the form of a pharmaceutically acceptable salt. Suitable salts are the hydrochloride, phosphate, succinate, maleate, theoclate, lactate, citrate and tannate.

The preferred antihistamine is diphenhydramine hydrochloride.

The antihistamine will be the active ingredient in a pharmaceutical composition. Examples of suitable pharmaceutical forms of capsules, tablets and liquids such as syrups. Typically, the antihistamine will be present in the composition in an amount of 20 to 100mg.

The pharmaceutical composition will be administered to a person after that person has consummed alcohol. If the composition is administered before the alcohol is taken, it might have a stimulatory effect.

Examples of suitable pharmaceutical compositions of the invention are set out below:

<u>Capsules</u>

| | |
|---|---|
| Diphenhydramine  HCl | 25mg |
| Paracetamol | 250mg |
| L-Carnitine | 300mg |
| Excipients | q.s. |

<u>Tablets</u>

| | | |
|---|---|---|
| Diphenhydramine  HCl | | 30mg |
| Paracetamol | | 350mg |
| Tablet excipients | up to | 550mg |

<u>Tablets</u>

| | | |
|---|---|---|
| Promethazine hydrochloride | | 25mg |
| Paracetamol | | 350mg |
| Tablet excipients | up to | 550mg. |

The diphenhydramine hydrochloride tablets set out above were evaluated in a double blind cross-over trial with placebos. Seventeen people participated in the trial and the dose administered was two tablets taken after the intake of alcohol. This trial showed conclusively that the tablet reduced the after-effects of over indulgence of alcohol.

**Claims**

1. An antihistamine for use in a method of manufacturing a composition for reducing the after-effects of over-indulgence of alcohol.

2. An antihistamine according to claim 1, wherein the composition is in a form suitable for oral administration.

3. An antihistamine according to claim 1 or 2, wherein the composition is in the form of capsules, tablets or a liquid.

4. An antihistamine according to any of claims 1 to 3, wherein the composition contains the antihistamine in an amount of 20 to 100mg.

5. An antihistamine according to any of claims 1 to 4, which is an ethanolamine, ethylenediamine, alkylamine, piperazine or phenothiazine.

6. An antihistamine according to any of claims 1 to 4, which is diphenhydramine, doxylamine, pyrilamine,

3

chlorpheniramine, chlorcyclizine or promethazine.

7. An antihistamine according to any of claims 1 to 6, which is in the form of a pharmaceutically acceptable salt.

8. An antihistamine according to any of claims 1 to 4, which is diphenhydramine hydrochloride.